# EUROPEAN PATENT SPECIFICATION

(11) **EP 3 190 410 B1**
(45) Date of publication and mention of the grant of the patent: **31.10.2018**
(21) Application number: 16020431.9
(22) Date of filing: 03.11.2016
(51) Int. Cl.: G01N 33/02, A23G 1/00

(54) **APPARATUS AND PROCESS FOR TESTING SAMPLES OF CONFECTIONERY MASS**
VORRICHTUNG UND VERFAHREN ZUR UNTERSUCHUNG VON PROBEN VON SÜSSWARENMASSE
APPAREIL ET PROCÉDÉ DE TEST D'ÉCHANTILLONS DE MASSE DE CONFISERIE

(43) Date of publication of application: 12.07.2017
(73) Proprietor: Aasted ApS, 3520 Farum (DK)
(72) Inventor: Holmud, Dennis, DK-2860 Søborg (DK)
(74) Representative: Heiden, Finn

(56) References cited:
- EP-A1- 2 085 774
- EP-A1- 2 319 328
- WO-A1-2008/119530
- AU-A- 1 359 395
- DE-U1-202011 100 655
- US-A1- 2005 241 491
- US-A1- 2011 174 077

## Description

The present invention concerns an apparatus for testing samples of tempered, liquid confectionery mass, such as tempered chocolate mass, comprising a receiving opening for a sample cup adapted to fit into the opening, tempering means adapted to cool or heat the sample cup, a lid adapted for fitting on top off the sample cup, a temperature measuring probe extending into the cup chamber at least when the lid is arranged on top of the cup.

The invention also relates to a process for testing a confectionery mass sample, such as tempered chocolate mass with the apparatus.

Such apparatuses are used within the field of manufacturing confectionery articles for obtaining the solidification curve of masses. The confectionery masses are typically chocolate mass and recipes having a content of cocoa butter mass or any substitutes such as palm kernel oil or other types of oil and milk fat and sugar. Masses such as creme mass for chocolate and bakery articles and fillings in pralines and enrobed articles. Masses such as in protein bars and other health bars being enrobed or encapsulated with mass such as chocolate. Marcipan masses and other masses with extremely high sugar content such as masses in Danish Bread and the like articles being enrobed with genuine chocolate.

Especially are the apparatuses known as temper meters being widely applied in the chocolate manufacturing industry for testing the condition of the liquid chocolate when it is ready tempered. This is before it is supplied to a moulding or enrobing line where the multiple chocolate articles solidify in a controlled environment. The cups are pressed out of aluminium blanks and their volume capacity are typically in the range of 50 ml.

The cooling means of the tempermeter apparatus is mostly arranged as a cool spot in an aluminium block. In the block is an opening into which a cup is inserted. The cool spot keeps an exact temperature of 8°C.

At a position in the manufacturing line just after the chocolate tempering apparatus is extracted a sample of liquid chocolate, which is dosed into a sample cup. The cup is inserted into the opening of the tempermeter apparatus. The lid is then fitted onto the top of the cup thereby simultaneously inserting the temperature measuring probe into the liquid chocolate. The chocolate sample temperature is thereafter logged continuously during cooling and solidification with time. During the logging period of typically 15 minutes the so-called "Temper curve" for the particular chocolate sample is disclosed progressively on the apparatus screen, may be printed and is simultaneously stored for later use.

The temper curve has for around 40 years been regarded as the most relevant tool for evaluating the "quality" of a tempered chocolate mass. Chocolate contains genuine cocoa butter, which is extracted from the cacao bean. Because of the nature of cocoa butter, real chocolate requires going through a tempering procedure, which re-establishes the cocoa butter crystals, providing the cooled and finished chocolate the proper shine, snap, taste, bloom resistance and storage ability.

All practical experience as well as scientific knowledge agrees that the BetaV form of cocoa fat crystals is the most desirable one to create in the tempered chocolate recipe. The size of the crystals should be as small as possible and they should be great in numbers. The solidified chocolate will then contract a small amount - typically 0,5-2% - which is necessary for the articles to leave moulding cavities without problems.

According to the manufacturers of the Temper Meter apparatuses, the slope reading of a particular "Temper curve" allows the user to know the degree in which the chocolate sample is "over"- or "under-tempered". By the term "over tempered" is obviously understood, that the chocolate sample contains too many crystals and by "under tempered" that the chocolate contains too few crystals.
This result is particularly useful to assist in achieving the best temper possible for the particular manufacturing purpose, thus providing all the benefits that come with properly tempered chocolate.

Most of the temper meters in the market use the slope of the temper curve for calculation of a tempering index. The scale varies between 1 and 9. "1" means very few crystals and "9" a lot of crystals. The best method is to use the slope as a reference for an optimal comparison of tempering values.

However, the slope of the temper curve is not the only useful information on the tempering quality. Also the temperature in the beginning of the crystallization is very important. It happens that different temperatures give the same slope and the same index.

Neither does the tempering curve give any information about the forms of the crystals. A much more sophisticated method such as the DSC measurement is necessary to determine the crystal forms in the sample. DSC apparatuses are seldom in the industry of manufacturing chocolate articles and are mostly used in laboratories researching in chocolate characteristics. They are very expensive and are requiring scientific knowledge to handle and evaluate results from.

So, the problematic is to provide an apparatus, which in a simple manner provides a certain and safe result for the common user who need not to be a professor in confectionery or chocolate behaviors. The result must directly reflect if the desirable quality of the mass sample is achieved or not.

It is well-known, that the desirable BetaV form crystals set the chocolate in a stable form, especially when being present in smallest sizes and in great amounts. The particular chocolate thereby demonstrates a desirable contraction during solidification as well. The inventor has followed this line of thinking being challenged by the problematic of providing an apparatus that in a simple manner will reveal the contraction of a confectionery sample, such as a chocolate sample. During intensive research he discovered, that higher and higher contraction of a particular chocolate is caused by higher and higher numbers of the BetaV crystals in the chocolate concomitant with the solidification of the BetaV crystals in as small size as possible. So when obtaining a "high" contraction of the tempered chocolate the desirable qualities of the BetaV content follows as gloss, superior taste, fine snap, bloom resistance and longest storage capability.

WO2009138246 discloses to oscillate the cavity walls of the moulds in a chocolate moulding line and measure the damping of these oscillations as the chocolate articles are solidifying.

EP2319328A1 discloses a simplified process for tempering cocoa butter containing mass whereby form VI crystals can be obtained. The temperature is decreased from an initial temperature of between 53°C and 57°C and to a final temperature of between 30 °C and 34°C in a single step by use of a tank equipped with a scraper and an impeller. The impeller induces a downward current of the cocoa butter containing mass in the tank. A pipe for product circulation during processing time leads from the bottom of the tank and returns to the top of the tank. At the pipe is arranged a device for measuring the pressure drop of the circulating chocolate mass and thus the viscosity of the mass, thereby enabling the assessment of the crystallization state of the cocoa butter containing mass.

US2005/241491A1 discloses an apparatus for obtaining crystallization solidification curves of chocolate mass in a continuous production of such mass. The apparatus includes a removal location, a measurement chamber and a melting chamber. The removal location is arranged to remove a liquid sample of chocolate mass from a flow of mass in a production tube. The sample is transferred to the location of a measurement chamber outside the flow. A temperature sensor is arranged in the measurement chamber and connected to a unit. During solidification of the mass sample the march of temperature is recorded by the unit and evaluated for determining crystallization and solidification curves in the known manner.

AU1359395A discloses a temperature probe arranged in a sample chamber, which is part of a cooling tube arranged in a cooling bucket. The probe is connected to a processor. A liquid chocolate sample is deposited in the sample chamber and the processor produces a cooling curve from the output signal from the probe when the sample is cooled and solidifies. The curve is evaluated for determining the degree of tempering of the sample.

The professional laboratory equipment manufacturer Netzsch offers a dilatometer apparatus in which the foodstuff sample is arranged in between to plates, the one is stationery and the other is free to move. The travel of the other plate is directly visible as a displacement of a liquid column in a glass tube as the food sample solidifies. The travel of the liquid column directly expresses the expansion or contraction of the sample. However, the dilatometer apparatus doesn't seem to have any spreading in the chocolate manufacturing industry. Instead a fine DSC apparatus from Netzsch is well applied.

A well applied method in the chocolate industry is to test the hardness of a chocolate sample. The hardness is regarded as synonymous with the contraction as high hardness corresponds to high contraction of the chocolate in the sample.

Cracks or ruptures in the surface of confectionery articles due to contraction of the mass in the center thereof are everyday experience for many consumers.
A problem to be solved is then how to make sure that a filling or center confectionery mass not will contract so much during solidification on the article, that the contraction initiates cracks or ruptures in an encapsulation such as a moulded chocolate shell or an enrobed layer.

Another problem is to make sure that the confectionery mass enrobing or encapsulating the center of the articles will not contract to the extent that cracks or ruptures are present.

A further problem is to make sure that the confectionery mass is controlled when production thereof is altered in terms of the tempering thereof.

A certain problem to be solved for chocolate mass with a content of genuine cocoa butter is to make sure that the desired quality of the mass is obtained.

The inventive apparatus is characterized in, that gasket means is arranged at the lid or at the cup adapted to close of the lid around the periphery of the cup thereby providing an airtight volume in the cup, that an opening is extending from the inside to the outside of the lid, and that a channel is connecting the opening with a pressure or volume measuring device. A sample of liquid chocolate is poured into the cup. The cup is arranged in the receiving opening and the lid is fitted on top of the cup closing off around the periphery of the lid. The cup and the chocolate is cooled by the cooling means whereby the chocolate sample solidifies and contracts.
The temperature of the solidifying chocolate sample is logged ongoing by the electronic calculation device or computer unit. Simultaneously are registered the alterations in pressure or volume caused by the contraction of the chocolate sample registered via the opening in the lid and the channel by the vacuum or volume measuring device.

The size of the pressure or of the volume change from liquid to solidified condition of the confectionery mass sample is a direct expression for the degree or percentage of contraction of the particular sample. Consequently, the result and degree of the contraction is directly obtainable. It may for example be disclosed on a screen so it can be used for straight forward evaluation of the particular chocolate sample and if its contraction and quality is acceptable for the intended use.

The inventive process is characterised in, that the liquid mass sample is deposited into a chamber volume thereby being filled out with liquid sample, which chamber is closed and made airtight, and that the mass sample is cooled or heated as the temperature of the sample is logged, and simultaneously is measured the increasing or decreasing pressure or volume change exerted by the sample.
The invention is explained further below under reference to preferred embodiments as well as the drawing, in which
fig. 1 is a schematic view of the inventive apparatus for testing chocolate samples, seen from the front,
fig. 2 is a vertical section at A in figure 1, seen from the side,
fig. 3a is a perspective view of a sample cup from the apparatus of figure 1 and 2,
fig. 3b is the same as in figure 3, seen in vertical section,
fig. 4a is a vertical section at B in figure 1, seen from the front, with the cup volume filled with a sample of liquid tempered chocolate ready for solidification and measuring,
fig. 4b is the same as in figure 4a with the cup volume filled with a sample of liquid tempered chocolate solidifying, seen in vertical section at A as in figure 2,
fig. 4c the same as in figure 4a when the chocolate sample has fully solidified,
fig. 5 is graphs of the contraction of two differently tempered chocolate samples during solidification thereof,
fig. 6a is graphs of the contraction of two other chocolate samples during solidification thereof,

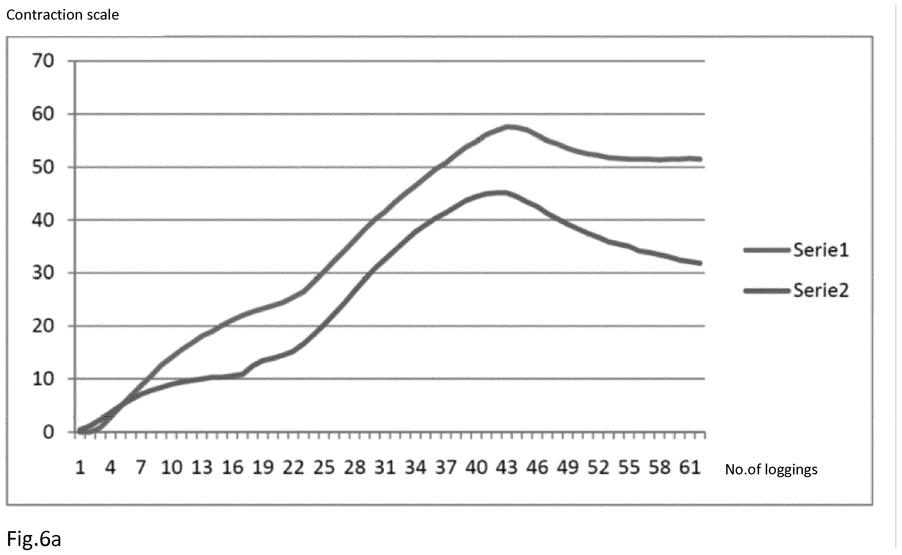

fig. 6a is graphs of the contraction of two different samples during solidification thereof, and

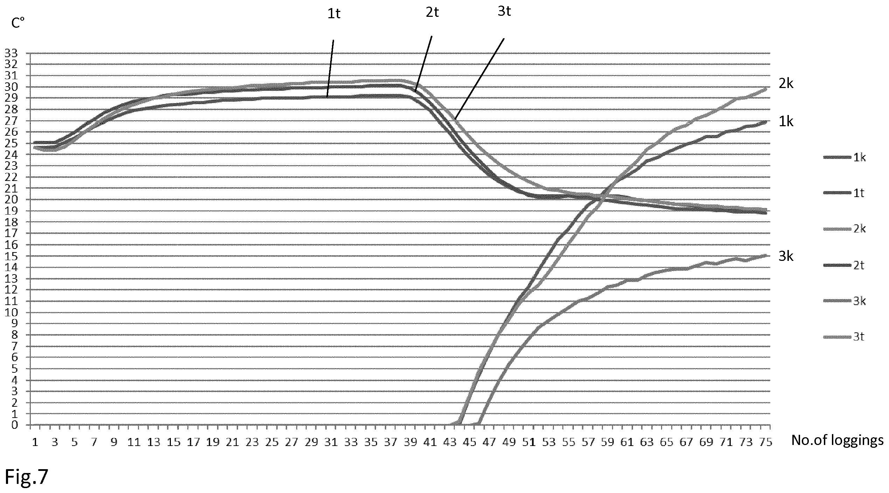

fig. 7 is graphs of the temperature and contraction of three samples of the same tempered chocolate mass, however being heated to different temperatures during the test for determining the crystal types in the mass and the melting points thereof.

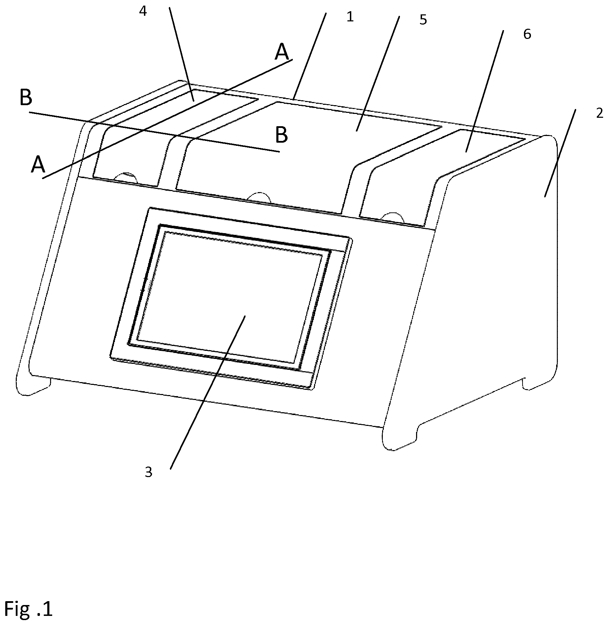

The apparatus 1 disclosed in figure 1 has a box-shaped casing 2 in which is arranged a digital touch-screen 3 as well as three lids 4, 5 and 6. The casing is typically made of a combination of plastic sheaves and milled aluminium-plates. In the casing is arranged a non-disclosed power source, typically 12 or 24 VDC for supplying the screen, the calculating source for the screen, or other calculator or computer device such as a PLC device that is capable of receiving measurements data, storing these and converting them to results such as digitally disclosed graphs at the screen 3.

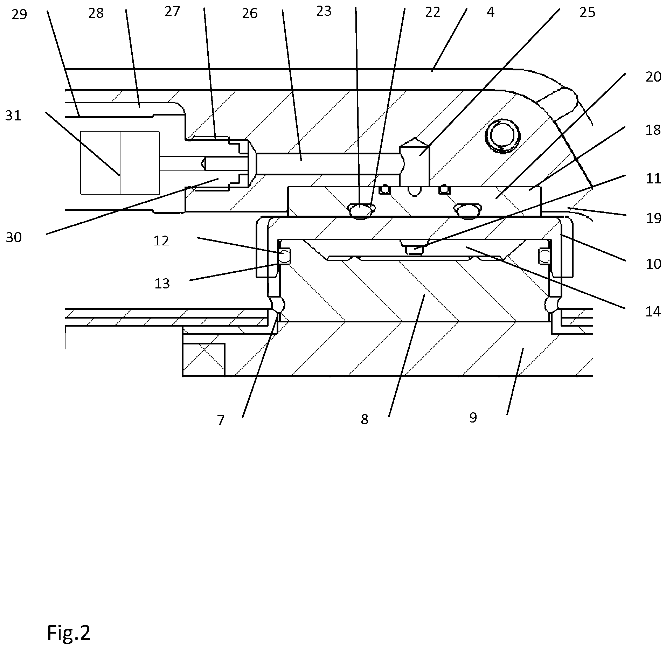

Under the lid 4 is arranged the inventive system as disclosed in figure 2. Under the other lids 5, 6 may be arranged a further inventive system or other systems traditionally applied in the testing of confectionery masses, such as a tempermeter system for logging the temperature of a solidifying sample of chocolate mass. Under the lids may also be space for storing sample cups or other auxiliary parts being used during testing of confectionery masses.

At figure 2 is disclosed the inventive system arranged under the lid 4 of the apparatus 1 in a closed position. A receiving opening 7 is arranged in the apparatus and is visible when the lid 4 is open. A sample cup 8 is adapted to fit into the opening 7. Cooling or heating means 9 is arranged under the opening 7 and is adapted to cool or heat up the cup 8 when it is inserted in the opening 7 as disclosed in figure 2. The cooling means is in figure 2 disclosed as a block 9 of aluminum being milled to the desired shape. The plate 9 is typically cooled by an electrically powered cooling unit, which is not disclosed as being common. When the polarity is reversed for the electrically powered unit, it is instead of cooling adapted to heat up the block 9.

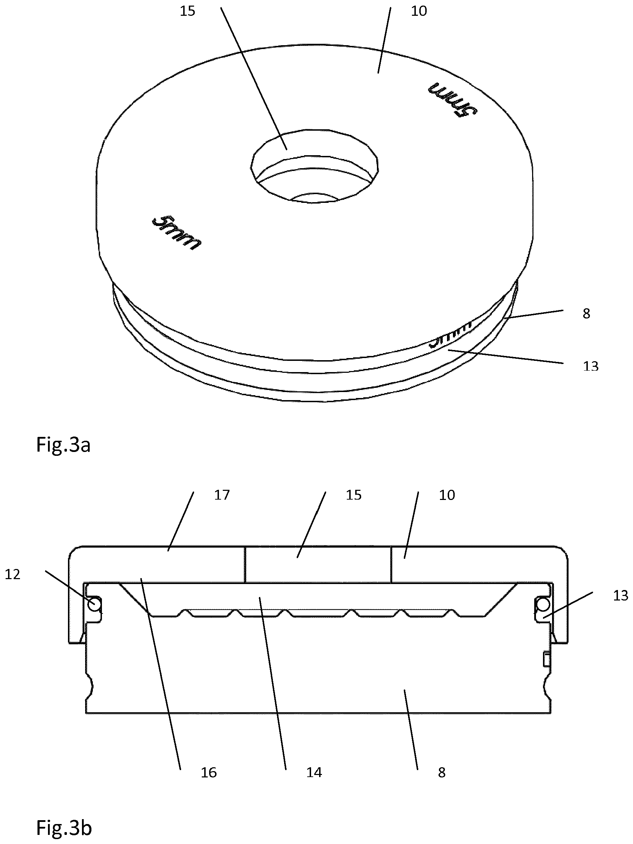

A lid 10 is adapted to fit tightly on top of the sample cup 8 as disclosed in figure 2 and in more detail in figure 3a and 3b. A temperature measuring probe 11 is extending into the chamber 14 of the cup 8 at least when the lid 4 is in the closed position and thereby being arranged on top of the cup 8.

The temperature measuring probe 11 is wired to an electronic calculation device or computer unit which could be implemented in the backside of the digital screen 2, or the screen could be wired to the computer unit. The connections could also be wire-less, however the electronic calculation device or computer unit is not disclosed as being common.

A gasket in the form of an O-ring 12 is arranged in a peripheral groove 13 in the cup 8 adapted to close of the lid 10 around the periphery of the cup 8 thereby providing an airtight chamber 14 for receiving the liquid mass sample.

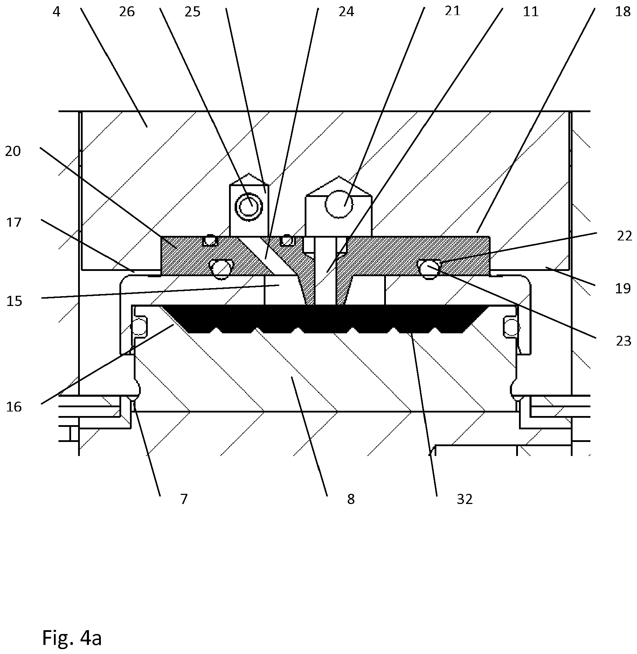

An opening 15 is extending from the inside 16 to the outside 17 of the lid 10 as depicted in figure 3a, 3b and 4a.

In a hole 18 at the underside 19 of the apparatus lid 4 is arranged a plastic sheave 20, which carry the centrally arranged temperature measuring probe 11. The sheave 20 has a central tap 21 which carries the probe 11 and ensures that the probe 11 extends into the airtight chamber 14 in closed position of the lid 4. The probe 11 then extends into the liquid mass sample and ensures a safe logging of the temperatures in the sample. Via the bore 21 may non-disclosed wires extend from the probe 11 and connect it with the electronic calculation device or computer unit.

In the plastic sheave 20 is arranged a circumferential groove 22, into which is arranged a gasket, typically an O-ring 23 which fits tightly on to the top of the lid 10 for the sample cup 8.

A channel 24 extends from inside the groove 22 and through the plastic sheave 20 to a further bore 25 in the lid 4. From the bore 25 extends a second channel 26 in the lid 4 to an enlarged hole 27 with thread as well as an even further enlarged hole 28. In the hole 28 is inserted a vacuum pressure measuring device 29, the end 30 of which is screwed into the thread of the hole 27. The vacuum pressure measuring device 29 is wired to a computer or calculator and the digital touch-screen. The wiring is not disclosed as the transfer of data could also be wire-less.

The vacuum pressure measuring device 29 has an inner membrane 31, which is disclosed in relaxed position in figure 2. As the channel 24 extends from the chamber 14 to the bore 25, and via the channel 26 leads directly to the membrane 31, then the airtight chamber 14 is closed off by the membrane 31. The flexible membrane 31 is therefore directly exposed to changes in the vacuum pressure or overpressure of the chamber 14 when the mass in the chamber is contracting or expanding.

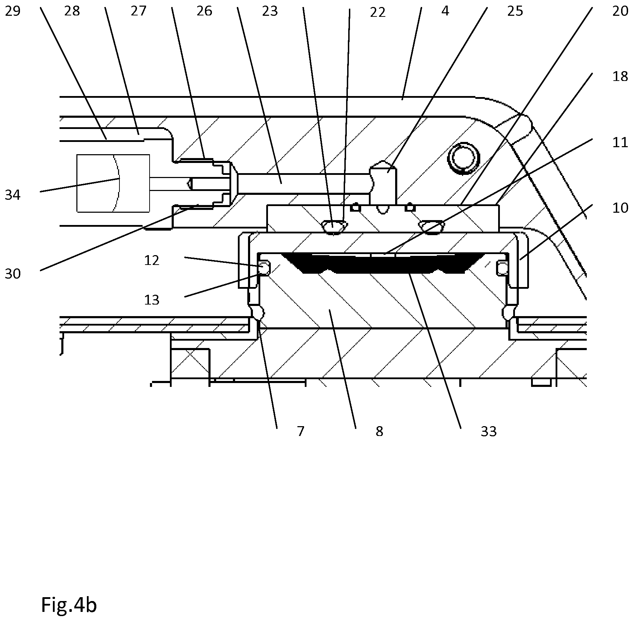

The vacuum pressure measuring device 29 is typically of the type at which a strain gauge element is arranged at the membrane 31. When the membrane 31 extends as disclosed in figure 4b the increasing vacuum pressure is measured as by the strain gauge as increasing tension force given in current as mA. The values of the strain gauge is in the range of 4-20 mA, which is transferred and calculated to mbar vacuum pressure or percentage contraction.

A test begins when an exact amount of the liquid confectionery mass is extracted from the production such as from the tempered chocolate mass. The exact amount of chocolate mass 32 is deposited into and fills out the chamber 14 of the sample cup 8. The lid 10 is placed securely on top of the cup 8 and the cup is arranged in the receiving opening 7 in the apparatus 1 as depicted in figure 4a.

The particular atmospheric pressure at the environments is obtained by the vacuum pressure measuring cylinder 29 and a nullification of the measuring device or the graphical disclosure of the results is performed before any logging of vacuum pressure or volume values begin.

The cooling block 9 is cooled and the logging of the increasing vacuum pressure exerted against the membrane 31 as obtained, received and displayed simultaneously at the screen 3 as the sample solidifies and contracts gradually.
Simultaneously is the temperature of the chocolate sample obtained and likewise displayed at the screen 3.

In figure 4b is disclosed the slight contraction of the chocolate, which now has obtained a smaller volume depicted as sample 33. Correspondingly herewith the membrane has extended and has obtained a larger shape as the extended membrane 34.

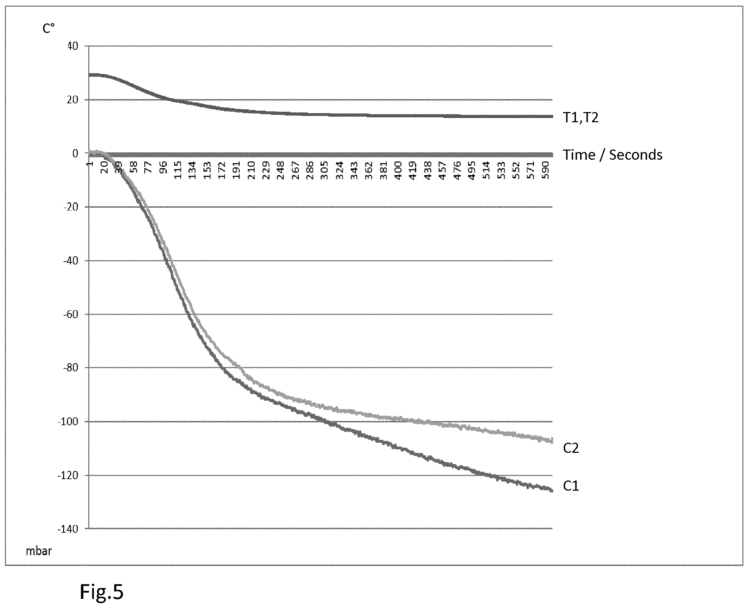

During the solidification the vacuum pressure is displayed directly as a graph at the screen in figure 5. The result of a first sample of tempered chocolate mass is disclosed as the graph C1. A further sample of the same chocolate mass, which however is differently tempered is disclosed as the graph C2. The temperatures logged during the solidification are represented by the graphs T1 and T2, which are identical.

The graphs of vacuum pressure directly represents the obtained contraction for the particular sample. The two graphs C1 and C2 therefore represents the degree of contraction of the two samples. It is immediately obtainable that the contraction of sample C1 is greater than the contraction of sample C2. Sample C1 is therefore desirable over C2 when it comes to the quality of tempered chocolate mass.

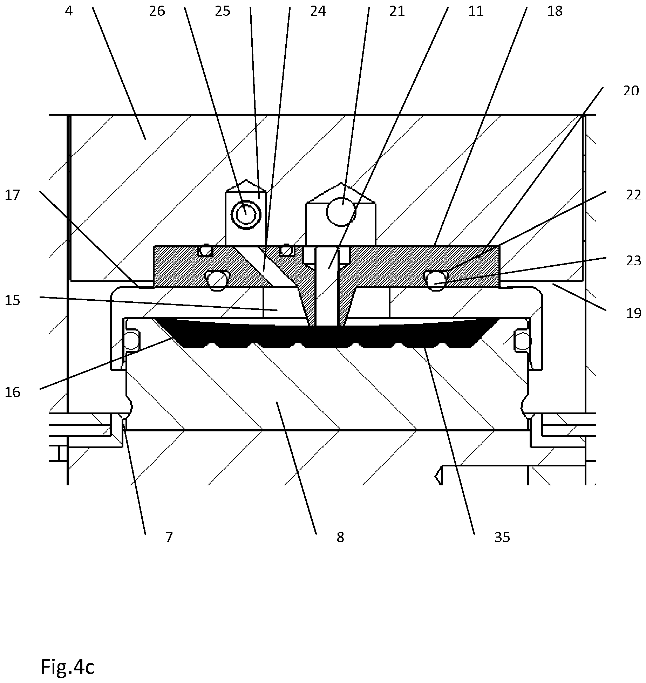

In figure 4c is disclosed the finally solidified chocolate sample 35. The contraction is disclosed exaggerated, as it normally is between 0-2% when it comes to tempered chocolate mass.

In figure 6a is disclosed the graphs representing two other samples of differently tempered chocolate masses, series 1 and series 2. The logged values of the contractions are feed into an equation, so that they are displayed at the "positive" side of zero. At the X-axis the value 70 represents a contraction value such as 2,0%. The series 1 chocolate sample has higher contraction than the series 2 sample and is desirable for chocolate moulding as the better contraction is preferred.

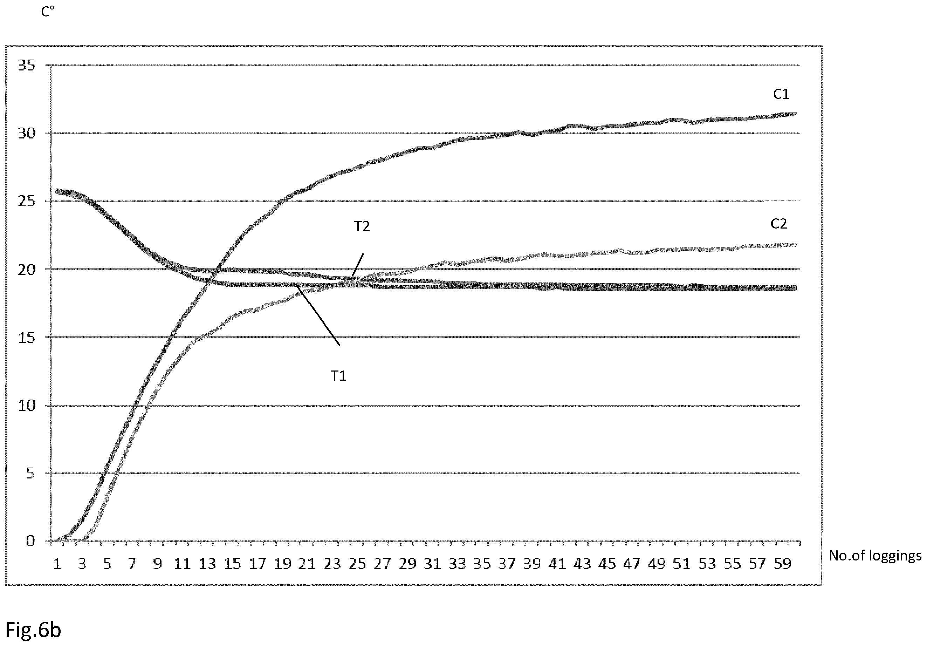

In figure 6b the graphs once more represent two differently tempered chocolate mass samples C1 and C2. The logged values of the contractions are converted to positive values before displayed. It is evident that the contraction of the sample C1 is bigger than that of the sample C2. The temperature graphs T1 and T2 for the samples differ some degrees during the solidification period.

Knowledge of the crystal melting point "CMP" of the crystals in a mass sample such as a liquid chocolate mass is valuable information, especially in the manufacturing of major amounts of consumer articles that have to stand up to their brand reputation. Quality is a must and with the new apparatus it's a simple and easy process to test a sample extracted from the production.

In figure 7 is disclosed the graphs of three logged temperature curves 1t, 2t and 3t as well as the respective contraction curves obtained 1k, 2k and 3k.

A sample of well-tempered liquid milk chocolate mass is extracted from a production line. The liquid sample is deposited into the sample cup 8 of the apparatus 1. The tempering block 9 is arranged for heating of the sample, which is heated to a first temperature of 29°C, i.e. the temperature curve 1t. The temperature of 29°C is maintained for at least one minute. The poles are inverted for the electrical tempering element of the block 9 so that it is able to cool instead of provide heating. The sample is then cooled to 18°C thereby solidifying and contracting as disclosed at the curve 1K. The temper curve 1t discloses to the skilled eye that a major content of crystals having a melting point higher than 29°C are present in the sample. It can be BetaV as well as Beta IV crystals. The corresponding contraction curve 1K supports this as the contraction is high and similar to the contraction obtained when the same sample is cooled directly to solidification without the heating in-between.

A second sample of the same liquid chocolate mass is tested in the same manner as before. It is then heated to a slightly higher temperature being 30°C and maintained for at least a minute. The poles are inverted and the second sample is cooled to 18°C thereby solidifying and contracting as disclosed at the curve 2K. The temper curve 2t deviates from the curve 1t of the first sample as well as the contraction curve 2K deviates from the first contraction curve 1K.

In conclusion, the second test has revealed a content of Beta IV crystals having a melting point between 29°C and 30°C, which were melted up in the sample.

A third sample of the same liquid mass is also tested in the same manner. It is heated to 31,5°C and maintained for at least a minute. The poles are inverted and the third sample is cooled to 18°C thereby solidifying and contracting as disclosed at the curve 3K. The temper curve 3t has a vanishing expression in terms of disclosing crystallization. Especially, in comparison with the curves 1t and 2t the fact is, that no crystals were present in the mass when it was cooled from 31,5°C. It is supported by the corresponding contraction curve 3K, which tells us that the contraction is reduced to approximately the half of the contraction at first and second samples. It means that the contraction caused by crystallization is vanished, left is only contraction caused by heat release when temperature drops. For tempered chocolate mass the contraction caused by the crystallization is typically 40-50% of the total contraction.
The major content of crystals in the tempered mass sample then has a melting temperature of between 30°C and 30,5°C.

In conclusion, the sample test has revealed, that the tested tempered milk chocolate has a major content of Beta V crystals with a melting temperature in the range of 30-30,5°C as well as a minor content of Beta IV crystals with a melting temperature in the range of 29-30°C.
- 1:: apparatus for testing samples of confectionery mass
- 2:: casing
- 3:: digital touch-screen
- 4:: lid
- 5:: lid
- 6:: lid
- 7:: receiving opening
- 8:: sample cup
- 9:: tempering block of aluminum
- 10:: lid for sample cup
- 11:: temperature measuring probe
- 12:: O-ring
- 13:: peripheral groove
- 14:: airtight chamber
- 15:: opening
- 16:: inside of lid
- 17:: outside of lid
- 18:: hole
- 19:: underside
- 20:: plastic sheave
- 21:: bore
- 22:: circumferential groove
- 23:: O-ring
- 24:: channel
- 25:: bore
- 26:: second channel
- 27:: enlarged hole
- 28:: further enlarged hole
- 29:: pressure measuring cylinder
- 30:: threaded end of cylinder
- 31:: membrane
- 32:: liquid sample of confectionery
- 33:: chocolate sample during solidification
- 34:: extended membrane
- 35:: finally solidified sample

## Claims

1. Apparatus (1) for testing samples (32,33,35) of tempered, liquid confectionery mass, such as tempered chocolate mass, comprising a receiving opening (7) for a sample cup (8) adapted to fit into the opening (7), tempering means (9) adapted to cool or heat the sample cup (8),
a lid (10) adapted to fit on top of the sample cup (8), a temperature measuring probe (11) extending into the cup chamber (14) at least when the lid (10) is arranged on top of the cup (8),
**characterised in,**
**that** gasket means (12) is arranged at the lid (10) and is adapted to close the lid (10) around the periphery (13) of the cup (10) thereby providing an airtight volume in the cup chamber (14), and wherein an opening (15) extends from the inside (16) to the outside (17) of the lid (10), and that a channel (24, 25, 26) is arranged to connect the opening (15) with a pressure or volume measuring device (29).

2. Apparatus according to claim 1, **characterised in, that** the pressure or volume measuring device (29) has a membrane (31) adapted to respond to changes in pressure or changes in volume during solidification or during melting of the confectionery mass sample (32,33,35).

3. Apparatus according to claim 1, **characterised in, that** a strain gauge is arranged at the membrane (31) adapted to detect the obtained values for changes in pressure or changes in volume during solidification or during melting of the confectionery mass sample (32,33,35).

4. Apparatus according to claim 1, **characterised in, that** the apparatus comprises an upper lid (4) in which the pressure measuring device (29) is arranged as well as the channel (24, 25, 26) connecting the opening (15) in the lid (10) with the pressure or volume measuring device (29).

5. Apparatus according to claim 1, **characterised in**, and that the upper lid (4) is adapted to arrest the lid (10) of the sample cup (8) with a secondary gasket (23) in between and around the opening (15) in the lid (10) of the cup (8).

6. Apparatus according to claim 1, **characterised in, that** tempering means (9) for cooling or heating is arranged under the cup (8) as support thereof.

7. Apparatus according to claim 1, **characterised in, that** the tempering means are thermoelectric, so that shifting between cooling and heating is adapted to be performed by polarity reversal.

8. Apparatus according to claim 1, **characterised in, that** the diameter of the airtight volume (14) in the cup is at least 10 times the height of the volume (14).

9. Process for testing a liquid confectionery mass sample (32), such as tempered chocolate mass, with the apparatus (1) of any of the claims 1-8, **characterised in, that** the liquid mass sample (32) is deposited into a chamber volume (14) thereby being filled out with liquid sample (32), which chamber (14) is closed and made airtight, and that the mass sample (32, 33) is cooled or heated as the temperature of the sample (33) is logged, and simultaneously measuring the increasing or decreasing pressure or volume change exerted by the sample (33, 35).

10. Process according to claim 9, **characterised in, that** the size of a partial volume no longer being filled out and growing as the sample solidifies and contracts, is measured as a value obtained from an extending membrane, which is exposed to the vacuum pressure.

11. Process according to claim 9, **characterised in, that** the size of the partial volume growing as the sample expands, is measured as a value obtained from an extending membrane, which is exposed to the increasing pressure.

12. Process according to claim 9, **characterised in, that** the particular atmospheric pressure is obtained and a nullification of measure devices are performed before any logging of pressure or volume values begin.

13. Process according to claim 9, **characterised in, that** the received values of pressure or volume are converted to values of percentage shrinkage or percentage expansion of the mass.

14. Process according to claim 9, **characterised in, that** the temperature of the mass sample is measured simultaneously with logging of the increasing vacuum pressure or volume change as the sample solidifies

15. Process according to claim 9, **characterised in, that** on the screen of an apparatus is depicted one curve for the values of contraction or expansion of the sample and simultaneously a second curve for the temperatures of the sample during the cooling or heating thereof.

16. Use of apparatus according to claim 1 for determining the contraction or expansion of a sample of confectionery mass during solidification or during heating.

17. Use of apparatus according to claim 1 for determining the contraction of a sample of chocolate mass during solidification.

18. Use of apparatus according to claim 1 for determining the expansion of a solidified sample of chocolate mass being heated.

19. Use of apparatus according to claim 1 for determining the contraction of a sample of creme mass during solidification.

20. Use of apparatus according to claim 1 for determining the contraction of a sample of center mass during solidification.

21. Use of apparatus according to claim 1 for determining the expansion of a sample of center mass during heating, which center mass has an outer casing of chocolate when ready manufactured.

22. Use of apparatus according to claim 1 for determining the melting temperatures of crystal content in confectionery mass, such as chocolate mass.

## Patentansprüche

1. Vorrichtung (1) zur Untersuchung von Proben (32, 33, 35) von temperierter flüssiger Süßwarenmasse, wie temperierter Schokoladenmasse, umfassend eine Aufnahmeöffnung (7) für eine Probenschale (8), die eingerichtet ist, um in die Öffnung (7) zu passen, Temperiermittel (9), die eingerichtet sind, um die Probenschale (8) zu kühlen oder zu erwärmen,
einen Deckel (10), der eingerichtet ist, um oben auf die Probenschale (8) zu passen,
eine Temperaturmesssonde (11), die sich mindestens dann, wenn der Deckel (10) oben auf der Schale (8) angeordnet ist, in die Schalenkammer (14) erstreckt,
**dadurch gekennzeichnet, dass**
ein Dichtungsmittel (12) an dem Deckel (10) angeordnet ist und eingerichtet ist zum Schließen des Deckels (10) um den Außenrand (13) der Schale (10) herum, wodurch ein luftdichtes Volumen in der Schalenkammer (14) bereitgestellt wird,
und wobei sich eine Öffnung (15) von der Innenseite (16) zu der Außenseite (17) des Deckels (10) erstreckt, und dass ein Kanal (24, 25, 26) angeordnet ist, um die Öffnung (15) mit einer Druck- oder Volumenmesseinrichtung (29) zu verbinden.

2. Vorrichtung nach Anspruch 1, **dadurch gekennzeichnet, dass** die Druck- oder Volumenmesseinrichtung (29) eine Membran (31) aufweist, die eingerichtet ist, um auf Änderungen des Drucks oder Änderungen des Volumens während des Erstarrens oder während des Schmelzens der Süßwarenmassenprobe (32, 33, 35) zu reagieren.

3. Vorrichtung nach Anspruch 1, **dadurch gekennzeichnet, dass** ein Dehnungsmessstreifen an der Membran (31) angeordnet ist, der eingerichtet ist, um die erhaltenen Werte für Änderungen des Drucks oder Änderungen des Volumens während des Erstarrens oder während des Schmelzens der Süßwarenmassenprobe (32, 33, 35) zu detektieren.

4. Vorrichtung nach Anspruch 1, **dadurch gekennzeichnet, dass** die Vorrichtung einen oberen Deckel (4), in dem die Druckmesseinrichtung (29) angeordnet ist, sowie den Kanal (24, 25, 26) umfasst, der die Öffnung (15) in dem Deckel (10) mit der Druck- oder Volumenmesseinrichtung (29) verbindet.

5. Vorrichtung nach Anspruch 1, **dadurch gekennzeichnet, dass** der obere Deckel (4) eingerichtet ist, um den Deckel (10) der Probenschale (8) mit einer Sekundärdichtung (23) zwischen der Öffnung (15) in dem Deckel (10) der Schale (8) und um diese herum zu arretieren.

6. Vorrichtung nach Anspruch 1, **dadurch gekennzeichnet, dass** Temperierungsmittel (9) zum Kühlen oder Erwärmen unter der Schale (8) angeordnet sind, um diese zu tragen.

7. Vorrichtung nach Anspruch 1, **dadurch gekennzeichnet, dass** die Temperierungsmittel thermoelektrisch sind, so dass Wechseln zwischen Kühlen und Erwärmen so eingerichtet ist, dass dies durch Polaritätsumkehr durchgeführt wird.

8. Vorrichtung nach Anspruch 1, **dadurch gekennzeichnet, dass** der Durchmesser des luftdichten Volumens (14) in der Schale mindestens das 10-fache der Höhe des Volumens (14) beträgt.

9. Verfahren zur Untersuchung einer flüssigen Süßwarenmassenprobe (32), wie temperierter Schokoladenmasse, mit der Vorrichtung (1) gemäß einem der Ansprüche 1 bis 8, **dadurch gekennzeichnet, dass** die flüssige Massenprobe (32) in einem Kammervolumen (14) abgesetzt wird, das dadurch mit flüssiger Probe (32) ausgefüllt wird, wobei die Kammer (14) verschlossen und luftdicht gemacht wird, und dass die Massenprobe (32, 33) gekühlt oder erwärmt wird, wobei die Temperatur der Probe (33) protokolliert wird, und gleichzeitig der zunehmende oder abnehmende Druck oder die zunehmende oder abnehmende Volumenänderung gemessen wird bzw. werden, der bzw. die durch die Probe (33, 35) ausgeübt wird.

10. Verfahren nach Anspruch 9, **dadurch gekennzeichnet, dass** die Größe eines Teilvolumens, das nicht länger ausgefüllt wird und wächst, wenn die Probe erstarrt und kontrahiert, als ein Wert gemessen wird, der von einer sich ausdehnenden Membran erhalten wird, die dem Vakuumdruck ausgesetzt ist.

11. Verfahren nach Anspruch 9, **dadurch gekennzeichnet, dass** die Größe des Teilvolumens, das wächst, wenn die Probe expandiert, als ein Wert gemessen wird, der von einer sich ausdehnenden Membran erhalten wird, die dem zunehmenden Druck ausgesetzt ist.

12. Verfahren nach Anspruch 9, **dadurch gekennzeichnet, dass** der jeweilige atmosphärische Druck erhalten wird und eine Nullung der Messeinrichtungen durchgeführt wird, bevor jedwedes Protokollieren von Druck- oder Volumenwerten beginnt.

13. Verfahren nach Anspruch 9, **dadurch gekennzeichnet, dass** die empfangenen Werte von Druck oder Volumen in Werte der prozentualen Schwindung oder prozentualen Expansion der Masse konvertiert werden.

14. Verfahren nach Anspruch 9, **dadurch gekennzeichnet, dass** die Temperatur der Massenprobe simultan unter Protokollierung des zunehmenden Vakuumdrucks oder der zunehmenden Volumenänderung gemessen wird, wenn die Probe erstarrt.

15. Verfahren nach Anspruch 9, **dadurch gekennzeichnet, dass** auf dem Bildschirm einer Vorrichtung eine Kurve für die Werte der Kontraktion oder Expansion der Probe und gleichzeitig eine zweite Kurve für die Temperaturen der Probe während des Kühlens oder Erwärmens derselben abgebildet wird.

16. Verwendung einer Vorrichtung nach Anspruch 1 zum Bestimmen der Kontraktion oder Expansion einer Probe von Süßwarenmasse während des Erstarrens oder während des Erwärmens.

17. Verwendung einer Vorrichtung nach Anspruch 1 zum Bestimmen der Kontraktion einer Probe von Schokoladenmasse während des Erstarrens.

18. Verwendung einer Vorrichtung nach Anspruch 1 zum Bestimmen der Expansion einer erstarrten Probe von Schokoladenmasse während des Erwärmens.

19. Verwendung einer Vorrichtung nach Anspruch 1 zum Bestimmen der Kontraktion einer Probe von Crememasse während des Erstarrens.

20. Verwendung einer Vorrichtung nach Anspruch 1 zum Bestimmen der Kontraktion einer Probe von Zentrumsmasse während des Erstarrens.

21. Verwendung der Vorrichtung nach Anspruch 1 zum Bestimmen der Expansion einer Probe von Zentrumsmasse während des Erwärmens, wobei die Zentrumsmasse eine Außenumhüllung aus Schokolade aufweist, wenn sie fertig hergestellt ist.

22. Verwendung der Vorrichtung nach Anspruch 1 zum Bestimmen der Schmelztemperaturen des Kristallgehalts in Süßwarenmasse, wie Schokoladenmasse.

## Revendications

1. Appareil (1) pour tester des échantillons (32, 33, 35) de masse de confiserie liquide tempérée, telle qu'une masse de chocolat tempéré, comprenant une ouverture de réception (7) pour une coupelle d'échantillon (8) adaptée pour s'ajuster dans l'ouverture (7), des moyens de tempérage (9) adaptés pour refroidir ou chauffer la coupelle d'échantillon (8),
un couvercle (10) étant adapté pour s'ajuster sur la partie supérieure de la coupelle d'échantillon (8),
une sonde de mesure de température (11) s'étendant dans la chambre de coupelle (14) au moins lorsque le couvercle (10) est agencé au-dessus de la coupelle (8),
**caractérisé en ce que**,
le moyen de joint d'étanchéité (12) est agencé au niveau du couvercle (10) et est adapté pour fermer le couvercle (10) autour de la périphérie (13) de la coupelle (10), fournissant ainsi un volume étanche à l'air dans la chambre de coupelle (14), et
une ouverture (15) s'étendant de l'intérieur (16) à l'extérieur (17) du couvercle (10), et un canal (24, 25, 26) étant agencé pour relier l'ouverture (15) à un dispositif de mesure de pression ou de volume (29).

2. Appareil selon la revendication 1, **caractérisé en ce que** le dispositif de mesure de pression ou de volume (29) a une membrane (31) adaptée pour réagir à des changements de pression ou des changements de volume pendant la solidification ou pendant la fusion de l'échantillon de masse de confiserie (32, 33, 35).

3. Appareil selon la revendication 1, **caractérisé en ce qu'**une jauge de contrainte est agencée au niveau de la membrane (31), adaptée pour détecter les valeurs obtenues pour des changements de pression ou des changements de volume pendant la solidification ou pendant la fusion de l'échantillon de masse de confiserie (32, 33, 35).

4. Appareil selon la revendication 1, **caractérisé en ce que** l'appareil comprend un couvercle supérieur (4) dans lequel le dispositif de mesure de pression (29) est agencé ainsi que le canal (24, 25, 26) reliant l'ouverture (15) dans le couvercle (10) au dispositif de mesure de pression ou de volume (29).

5. Appareil selon la revendication 1, **caractérisé en ce que** le couvercle supérieur (4) est adapté pour retenir le couvercle (10) de la coupelle d'échantillon (8) avec un joint d'étanchéité secondaire (23) entre et autour de l'ouverture (15) dans le couvercle (10) de la coupelle (8).

6. Appareil selon la revendication 1, **caractérisé en ce que** des moyens de tempérage (9) pour le refroidissement ou le chauffage sont agencés sous la coupelle (8) en tant que support de celle-ci.

7. Appareil selon la revendication 1, **caractérisé en ce que** les moyens de tempérage sont thermoélectriques, de sorte que le passage entre le refroidissement et le chauffage est adapté pour être effectué par inversion de polarité.

8. Appareil selon la revendication 1, **caractérisé en ce que** le diamètre du volume étanche à l'air (14) dans la coupelle est au moins 10 fois la hauteur du volume (14).

9. Procédé de test d'un échantillon de masse de confiserie liquide (32), tel qu'une masse de chocolat tempéré, avec l'appareil (1) selon l'une quelconque des revendications 1 à 8, **caractérisé en ce que** l'échantillon de masse liquide (32) est déposé dans un volume de chambre (14), rempli ainsi avec l'échantillon liquide (32), laquelle chambre (14) est fermée et rendue étanche à l'air, et **en ce que** l'échantillon de masse (32, 33) est refroidi ou chauffé alors que la température de l'échantillon (33) est enregistrée, tout en mesurant simultanément l'augmentation ou la diminution de la pression ou le changement de volume subi par l'échantillon (33, 35).

10. Procédé selon la revendication 9, **caractérisé en ce que** la taille d'un volume partiel n'étant plus remplie et croissant à mesure que l'échantillon se solidifie et se contracte, est mesurée en tant que valeur obtenue à partir d'une membrane d'extension, qui est exposée à la pression de vide.

11. Procédé selon la revendication 9, **caractérisé en ce que** la taille du volume partiel croissant à mesure que l'échantillon se dilate, est mesurée en tant que valeur obtenue à partir d'une membrane d'extension, qui est exposée à la pression croissante.

12. Procédé selon la revendication 9, **caractérisé en ce que** la pression atmosphérique particulière est obtenue et qu'une annulation des dispositifs de mesure est effectuée avant que n'importe quelle enregistrement de valeurs de pression ou de volume commence.

13. Procédé selon la revendication 9, **caractérisé en ce que** les valeurs de pression ou de volume reçues sont converties en valeurs de pourcentage de retrait ou de pourcentage d'expansion de la masse

14. Procédé selon la revendication 9, **caractérisé en ce que** la température de l'échantillon de masse est mesurée simultanément avec l'enregistrement de l'augmentation de la pression de vide ou du changement de volume lorsque l'échantillon se solidifie.

15. Procédé selon la revendication 9, **caractérisé en ce que**, sur l'écran d'un appareil, est représentée une courbe pour les valeurs de contraction ou d'expansion de l'échantillon, et simultanément une seconde courbe pour les températures de l'échantillon pendant le refroidissement ou le chauffage de celui-ci.

16. Utilisation de l'appareil selon la revendication 1, pour déterminer la contraction ou l'expansion d'un échantillon de masse de confiserie pendant la solidification ou pendant le chauffage.

17. Utilisation de l'appareil selon la revendication 1, pour déterminer la contraction d'un échantillon de masse de chocolat pendant la solidification.

18. Utilisation de l'appareil selon la revendication 1, pour déterminer l'expansion d'un échantillon solidifié de masse de chocolat chauffé.

19. Utilisation de l'appareil selon la revendication 1, pour déterminer la contraction d'un échantillon de masse de crème pendant la solidification.

20. Utilisation de l'appareil selon la revendication 1, pour déterminer la contraction d'un échantillon de masse centrale pendant la solidification.

21. Utilisation de l'appareil selon la revendication 1, pour déterminer l'expansion d'un échantillon de masse centrale pendant le chauffage, ladite masse centrale ayant une enveloppe externe de chocolat lorsqu'elle est prête à être fabriquée.

22. Utilisation de l'appareil selon la revendication 1, pour déterminer les températures de fusion de la teneur en cristaux dans une masse de confiserie, telle qu'une masse de chocolat.
